(19) Europäisches Patentamt
European Patent Office
Office européen des brevets

(11) Veröffentlichungsnummer: **0 583 435 B1**

# (12) EUROPÄISCHE PATENTSCHRIFT

(45) Veröffentlichungstag der Patentschrift: **23.08.95**

(51) Int. Cl.[6]: **A61K 7/13**, C07C 211/52, C07C 215/16

(21) Anmeldenummer: **93902146.5**

(22) Anmeldetag: **07.01.93**

(86) Internationale Anmeldenummer: **PCT/EP93/00011**

(87) Internationale Veröffentlichungsnummer: **WO 93/16677 (02.09.93 93/21)**

## (54) MITTEL ZUR OXIDATIVEN FÄRBUNG VON HAAREN UND NEUE 5-HALOGEN-2,4-BIS(ALKYLAMINO)-1-ALKYLBENZOLE.

(30) Priorität: **29.02.92 DE 4206416**

(43) Veröffentlichungstag der Anmeldung: **23.02.94 Patentblatt 94/08**

(45) Bekanntmachung des Hinweises auf die Patenterteilung: **23.08.95 Patentblatt 95/34**

(84) Benannte Vertragsstaaten: **DE ES FR GB IT**

(56) Entgegenhaltungen:
**WO-A-92/04005**
**DE-A- 1 937 230**
**DE-A- 3 724 642**
**FR-A- 2 112 550**
**GB-A- 2 176 494**

(73) Patentinhaber: **Wella Aktiengesellschaft**
**Berliner Allee 65**
**D-64295 Darmstadt (DE)**

(72) Erfinder: **BALZER, Wolfgang, R.**
**Schlesier Strasse 9a**
**D-6146 Alsbach (DE)**
Erfinder: **CLAUSEN, Thomas**
**Ernst-Pasque-Strasse 35a**
**D-6146 Alsbach (DE)**
Erfinder: **FRANK, Anke**
**Alte Falterstrasse 29**
**D-6230 Frankturt 80 (DE)**
Erfinder: **WEINGES, Alexa**
**Langgewann 41**
**D-6900 Heidelberg (DE)**

EP 0 583 435 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein Mittel zur oxidativen Färbung von Haaren auf der Basis von 5-Halogen-2,4-bis(alkylamino)-1-alkylbenzolen als Kupplersubstanzen sowie neue 5-Halogen-2,4-bis-(alkylamino)-1-alkylbenzole.

In der Haarfärbepraxis haben Oxidationsfarbstoffe eine wesentliche Bedeutung erlangt. Hierbei werden die Farbstoffe durch oxidative Kupplung von Entwicklersubstanzen und Kupplersubstanzen im Haarschaft erzeugt. Dies führt zu sehr intensiven Haarfärbungen mit sehr guter Farbechtheit. Außerdem können durch die Kombination geeigneter Entwickler- und Kupplersubstanzen unterschiedliche Farbnuancen erzeugt werden.

Als Entwicklersubstanzen werden bevorzugt 2,5-Diaminotoluol, 1,4-Diaminobenzol, 2-(2'-Hydroxyethyl)-1,4-diaminobenzol, 4-Aminophenol, 4-Amino-2-aminomethylphenol und 4-Amino-3-methylphenol verwendet.

Die bevorzugt verwendeten Kupplersubstanzen sind dabei m-Phenylendiamin und dessen Derivate, wie zum Beispiel 2,4-Diamino-phenoxyethanol, 2,4-Diaminobenzylalkohol, 2-Amino-4-(2'-hydroxyethyl)-aminoanisol oder Pyridinderivate wie 3,5-Diamino-2,6-dimethoxypyridin als Blaukuppler, 1-Naphthol, m-Aminophenol und dessen Derivate, wie 2-Amino-4-chlor-6-methylphenol, 3-Amino-2-methylphenol, 4-Amino-2-hydroxyphenoxyethanol und 3-Amino-5-hydroxy-2,6-dimethoxypyridin, als Rotkuppler sowie Resorcin, 4-Chlorresorcin, 4,6-Dichlorresorcin, 2-Methylresorcin, 4-Hydroxy-1,2-methylendioxybenzol, 4-(2'-Hydroxyethyl)amino-1,2-methylendioxybenzol und 4-Hydroxyindol als Kuppler für den Braun-Blond-Bereich.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwickler- und Kupplerkomponenten eine breite Palette verschiedener Farbnuancen erzeugt werden kann.

Die zur Zeit in Haarfärbemitteln zur Erzeugung insbesondere von roten und klaren blauen Farbtönen verwendeten Kupplersubstanzen können die vorstehend genannten Anforderungen jedoch nicht zufriedenstellend erfüllen.

Das aus der DE-OS 34 30 513 bekannte 2,4-Diamino-5-tetrafluorethoxytoluol zeigt zwar im Amestest keine mutagene Wirkung, jedoch sind Farbtiefe und Lichtechtheit der mit dieser Kupplersubstanz erzielten Ausfärbungen nicht zufriedenstellend.

Die in der DE-OS 36 22 784 beschriebenen Kupplersubstanzen 2,4-Diamino-5-ethoxytoluol beziehungsweise 2,4-Diamino-5-(2'-hydroxyethyl)oxytoluol besitzen zwar gute toxikologische Eigenschaften, zeigen jedoch mit Entwicklern wie p-Aminophenol oder dessen Derivaten nur sehr farbschwache Anfärbungen.

Demgemäß bestand die Aufgabe, ein Haarfärbemittel zur oxidativen Färbung von Haaren auf der Basis von in der Haarfärbung üblichen Entwicklersubstanzen mit einem Gehalt an neuen Kupplersubstanzen zur Verfügung zu stellen, bei dem die zuvor genannten gestellten Anforderungen an die anwendungstechnischen Eigenschaften der neuen Kupplersubstanz erfüllt werden.

Hierzu wurde nun gefunden, daß ein Mittel zur oxidativen Färbung von Haaren auf der Basis einer Entwicklersubstanz-Kupplersubstanz-Kombination sowie gegebenenfalls anderer Farbkomponenten und in Haarfärbemitteln üblichen Zusätzen, welches mindestens eine Kupplersubstanz der allgemeinen Formel

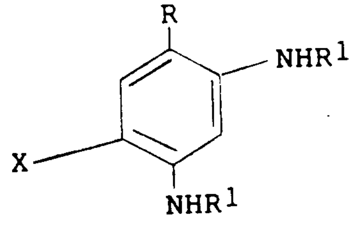

(I),

wobei R einen geradkettigen oder verzweigten $C_1$- bis $C_4$-Alkylrest, X Chlor oder Fluor und $R^1$ einen geradkettigen oder verzweigten $C_1$- bis $C_4$-Alkylrest oder einen geradkettigen oder verzweigten $C_2$- bis $C_4$-Hydroxyalkylrest darstellt, oder deren physiologisch verträgliches wasserlösliches Salz enthält, die gestellte Aufgabe in hervorragendem Maß löst.

Die Kupplersubstanzen der allgemeinen Formel (I) sind durch einfache chemische Reaktionen aus technisch zugänglichen Ausgangsverbindungen erhältlich und ergeben mit Entwicklersubstanzen wie p-Phenylendiamin klare blaue Ausfärbungen ohne Rotstich. Mit Entwicklersubstanzen vom Typ des p-Aminophenol lassen sich farbsatte, rote Färbungen erzielen, deren Farbintensität sogar größer ist als bei Ausfärbungen mit dem unsubstituierten 2,4-Diaminotoluol.

Die Kupplersubstanzen der allgemeinen Formel (I) sind gut in Wasser löslich und weisen, insbesondere als Bestandteil der hier beschriebenen Haarfärbemittel, eine ausgezeichnete Lagerstabilität auf.

In dem hier beschriebenen Haarfärbemittel sollen die erfindungsgemäßen Kupplersubstanzen der Formel (I), in einer für die Haarfärbung ausreichenden Menge, vorzugsweise in einer Menge von 0,01 bis 5 Gewichtsprozent, besonders bevorzugt 0,1 bis 3 Gewichtsprozent, enthalten sein.

Von den Kupplersubstanzen der allgemeinen Formel (I) sind in dem Haarfärbemittel bevorzugt 5-Fluor-2,4-bis(methylamino)-toluol,5-Fluor-2,4-bis(ethylamino)-toluol, 5-Fluor-2,4-bis[(2'-hydroxyethyl)amino]-toluol und 5-Chlor-2,4-bis[(2'-hydroxyethyl)amino]-toluol enthalten.

Außerdem können in dem Haarfärbemittel zusätzlich 0,01 bis 5 Gewichtsprozent, bevorzugt 0,1 bis 3 Gewichtsprozent, mindestens einer weiteren Kupplersubstanz, beispielsweise Resorcin, 4-Chlorresorcin, 4,6-Dichlorresorcin, 2-Methylresorcin, 2-Amino-4-(2'hydroxyethylamino)anisol, 2-Amino-4-ethylamino-anisol, 2,4-Diaminobenzylalkohol, m-Phenylendiamin, 5-Amino-2-methylphenol, 2,4-Diamino-phenoxyethanol, 1-Naphthol, m-Aminophenol, 3-Amino-4-chlor-6-methylphenol, 3-Amino-2-methylphenol, 4-Amino-2-hydroxyphenoxyethanol, 4-Hydroxy-1,2-methylendioxybenzol, 4-(2'-Hydroxyethylamino)-1,2-methylendioxybenzol, 2,4-Diamino-5-ethoxytoluol, 4-Hydroxyindol, 3-Amino-5-hydroxy-2,6-dimethoxypyridin und 3,5-Diamino-2,6-dimethoxypyridin, enthalten sein.

Von den Entwicklersubstanzen kommen als Bestandteil der erfindungsgemäßen Haarfärbemittel vor allem 1,4-Diaminobenzol, 2,5-Diaminotoluol, 2,5-Diaminobenzylalkohol, 2-(2'-Hydroxyethyl)-1,4-diaminobenzol, 4-Aminophenol, 4-Amino-2-aminomethyl-phenol, 4-Amino-3-methylphenol, 4-Amino-2-methoxymethylphenol, 4-Amino-2-ethoxymethylphenol sowie Tetraaminopyrimidin oder deren physiologisch verträglichen Salze in Betracht. Die in dem erfindungsgemäßen Mittel enthaltene Menge der Entwicklersubstanzen soll vorzugsweise 0,01 bis 5 Gewichtsprozent, besonders bevorzugt jedoch 0,1 bis 3,0 Gewichtsprozent, betragen.

Die üblichen Kuppler- und Entwicklersubstanzen können in dem erfindungsgemäßen Haarfärbemittel jeweils einzeln oder im Gemisch miteinander enthalten sein. Die Gesamtmenge der in dem hier beschriebenen Haarfärbemittel enthaltenen Entwicklersubstanz-Kupplersubstanz-Kombination soll etwa 0,1 bis 5,0 Gewichtsprozent, vorzugsweise 0,5 bis 4,0 Gewichtsprozent, betragen.

Die Entwicklersubstanzen werden im allgemeinen in etwa äquimolaren Mengen, bezogen auf die Kupplersubstanzen, eingesetzt. Es ist jedoch nicht nachteilig, wenn die Entwicklersubstanzen diesbezüglich in einem gewissen Überschuß oder Unterschuß vorhanden ist.

Weiterhin kann das Haarfärbemittel dieser Anmeldung zusätzlich auch andere Farbkomponenten, beispielsweise 6-Amino-2-methylphenol und 2-Amino-5-methylphenol sowie ferner übliche direktziehende Farbstoffe, zum Beispiel Triphenylmethanfarbstoffe wie Basic Violet 14 (C. I. 42 510) und Basic Violet 2 (C. I. 42 520), aromatische Nitrofarbstoffe wie 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethylamino)nitrobenzol und 4-(2'-Hydroxyethylamino)-3-nitro toluol, 1-(2'-Ureidoethyl)amino-4-nitrobenzol und Azofarbstoffe wie Acid Brown 4 (C. I. 14 805) sowie Dispersionsfarbstoffe, wie beispielsweise 1,4-Diaminoanthrachinon und 1,4,5,8-Tetraaminoanthrachinon, enthalten.

Weitere geeignete direkt auf das Haar aufziehende Farbstoffe sind beispielsweise im Buch von J. C. Johnson "Hair Dyes", Noyes Data Corp., Park Ridge, USA (1973), Seiten 3 - 91 und 113 - 139 (ISBN: 0-8155-0477-2) beschrieben.

Selbstverständlich können die Kuppler- und Entwicklersubstanzen sowie die anderen Farbkomponenten, sofern es Basen sind, auch in Form der physiologisch verträglichen Säureadditionssalze, wie beispielsweise als Hydrochlorid beziehungsweise Sulfat oder - sofern sie aromatische OH-Gruppen besitzen - in Form der Salze mit Basen, zum Beispiel als Alkaliphenolate, eingesetzt werden.

Das Haarfärbemittel kann ferner direktziehende Farbstoffe und mit sich selbst kuppelnde Farbstoffvorstufen in einer Menge von 0,1 bis 4,0 Gewichtsprozent enthalten.

Darüber hinaus können in dem Haarfärbemittel noch übliche kosmetische Zusätze, beispielsweise Antioxidantien wie Ascorbinsäure, Thioglykolsäure oder Natriumsulfit, Parfümöle, Komplexbildner, Netzmittel, Emulgatoren, Verdicker, Pflegestoffe und andere vorhanden sein.

Die Zubereitungsform kann beispielsweise eine Lösung, insbesondere eine wäßrige-alkholische Lösung, sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen

üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Haarfärbemittel schwach sauer, neutral oder alkalisch reagieren. Insbesondere weist es einen pH-Wert im alkalischen Bereich zwischen 8,0 bis 11,5 auf, wobei die Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid, Verwendung finden.

Für die Anwendung zur oxidativen Färbung von Haaren vermischt man das vorstehend beschriebenen Haarfärbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin oder Natriumborat in Form der 3 bis 12 %igen, vorzugsweise 6 %igen wäßrigen Lösungen, in Betracht.

Wird eine 6 %ige Wasserstoffperoxidlösung als Oxidationsmittel verwendet, so beträgt das Gewichtsverhältnis zwischen Haarfärbemittel und Oxidationsmittel 5 : 1 bis 1 : 2, vorzugsweise jedoch 1 : 1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet.

Man läßt das Gemisch bei 15 bis 50 °C etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Haarfärbemittel führt zu Haarfärbungen mit ausgezeichneten Echtheitseigenschaften, insbesondere was die Licht-, Wasch- und Reibechtheit anbetrifft, und läßt sich mit Reduktionsmitteln wieder abziehen. Hinsichtlich der färberischen Möglichkeiten bietet das erfindungsgemäße Haarfärbemittel je nach Art und Zusammensetzung der Farkomponenten eine breite Palette verschiedener Farbnuancen. Bemerkenswert ist die große Farbintensität der erzielbaren roten und die Farbreinheit der erzielbaren blauen Färbungen. Schließlich ist mit Hilfe des beschriebenen Haarfärbemittels auch eine Anfärbung von ergrauten und chemisch nicht vorgeschädigtem Haar problemlos und mit sehr guter Deckkraft möglich. Die dabei erhaltenen Färbungen sind, unabhängig von der unterschiedlichen Struktur des Haares, gleichmäßig und sehr gut reproduzierbar.

Die 5-Halogen-2,4-bis(alkylamino)-1-alkylbenzole der allgemeinen Formel (I) lassen sich einfach aus den entsprechenden technisch verfügbaren 3-Halogen-alkylbenzolen herstellen. In zwei Syntheseschritten, durch Nitrierung in 2,4 Stellung und nachfolgende Reduktion der Nitrogruppen, wird zunächst das entsprechende 5-Halogen-2,4-diamino-alkylbenzol (IV) gemäß dem nachstehenden Schema hergestellt, wobei R und X die vorstehend angegebene Bedeutung haben:

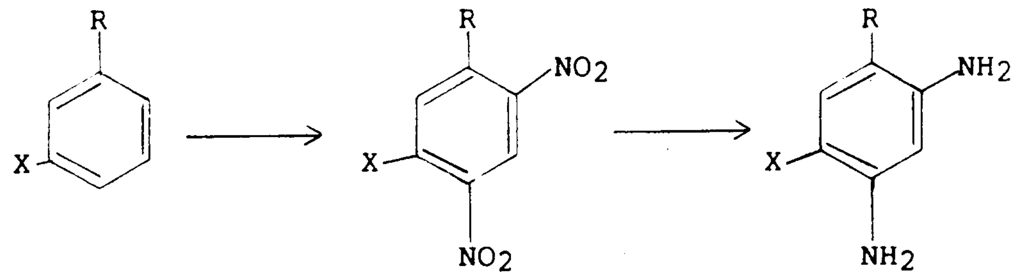

(II) (III) (IV)

Für die Nitrierung kommen die in der organischen Synthese üblichen Nitrierungsreagenzien in Frage, wobei ein Gemisch aus Schwefelsäure und Salpetersäure bevorzugt ist. Für die Reduktion der Nitrogruppen kommen ebenfalls die für diesen Syntheseschritt bekannten Reagenzien, beispielsweise Wasserstoff in Gegenwart eines geeigneten Katalysators, in Betracht.

Ausgehend von dem 5-Halogen-2,4-diamino-alkylbenzol (IV) sind die neuen Kupplersubstanzen der allgemeinen Formel (I) auf zwei Wegen zugänglich.

1. Das 5-Halogen-2,4-diamino-alkylbenzol (IV) wird mit dem entsprechenden $C_2$- bis $C_4$-Chlorameisensäurechloralkylester zu den hydroxyalkylierten erfindungsgemäßen Verbindungen der allgemeinen Formel (Ia), wobei $R^2$ einen geradkettigen oder verzweigten $C_2$- bis $C_4$-Hydroxyalkylrest darstellt und R und X die vorstehend angegebene Bedeutung haben, umgesetzt:

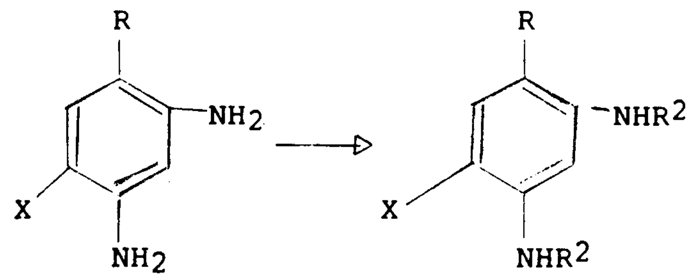

(IV) (Ia)

2. Die erfindungsgemäßen Verbindungen der Formel (Ib), bei denen die Aminogruppen mit einem geradkettigen oder verzweigten $C_1$- bis $C_4$-Alkylrest substituiert sind, lassen sich aus den entsprechenden 5-Halogen-2,4-diamino-alkylbenzolen (IV) in drei Syntheseschritten gemäß dem nachfolgend angegebenen Schema, durch Umsetzung mit einem für Aminogruppen üblichen Schutzgruppenreagenz, beispielsweise Benzolsulfonylchlorid, Alkylierung mit einem üblichen Alkylierungsreagenz, beispielsweise $C_1$- bis $C_4$-Alkyljodiden, und anschließende Entfernung der Schutzgruppen, wobei $R^3$ einen geradkettigen oder verzweigten $C_1$-bis $C_4$-Alkylrest und Y eine für Aminogruppen übliche Schutzgruppe darstellt und R und X die vorstehend angegebene Bedeutung haben, herstellen:

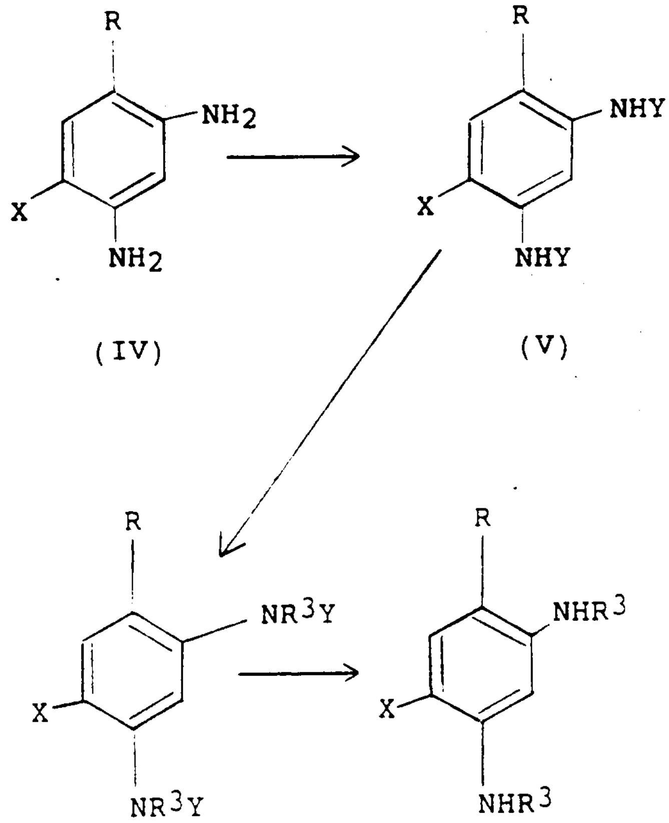

(IV) (V)

(VI) (Ib)

Gegenstand der vorliegenden Anmeldung ist ferner ein neues 5-Halogen-2,4-bis(alkylamino)-1-alkylbenzol der allgemeinen Formel

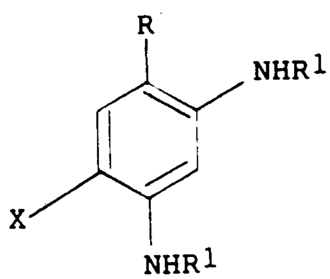

(I),

wobei R einen geradkettigen oder verzweigten $C_1$- bis $C_4$-Alkylrest darstellt, X die Bedeutung Chlor oder Fluor hat und $R^1$ einen geradkettigen oder einen verzweigten $C_1$- bis $C_4$-Alkylrest oder einen geradkettigen oder verzweigten $C_2$- bis $C_4$-Hydroxyalkylrest darstellt.

Als Beispiele für die neuen Verbindungen der Formel (I) seien 5-Fluor-2,4-bis(methylamino)-toluol, 5-Fluor-2,4-bis-(ethylamino)-toluol, 5-Fluor-2,4-bis[(2'-Hydroxyethyl)amino]-toluol und 5-Chlor-2,4-bis[(2'-hydroxyethyl)amino]-toluol genannt.

Die nachfolgenden Beispiele sollen den Gegenstand der Erfindung näher erläutern.

## Beispiele

### Beispiel 1: Haarfärbemittel in Gelform

0,22 g 5-Fluor-2,4-bis(methylamino)-toluol
1,10 g 2-(2'-Hydroxyethyl)-1,4-diaminobenzolsulfat
0,20 g Resorcin
0,05 g m-Aminophenol
0,40 g Natriumsulfit, wasserfrei
15,00 g Ölsäure
7,00 g Isopropanol
10,00 g Ammoniak (22 %ige wäßrige Lösung)
66,03 g Wasser
100,00 g

Man vermischt kurz vor der Anwendung 50 g dieses Haarfärbemittels in Gelform mit 50 ml einer 6 %igen Wasserstoffperoxidlösung. Das Gemisch wird sodann auf blonde menschliche Haare aufgetragen. Nach einer Einwirkungszeit von dreißig Minuten bei 40 Grad Celsius wird das Haar mit Wasser gespült und sodann getrocknet. Das Haar hat eine natürliche mittelblonde Färbung angenommen.

### Beispiel 2: Haarfärbemittel in Gelform

0,27 g 5-Fluor-bis(ethylamino)-toluol
2,80 g 2-Methyl-p-phenylendiamin
0,12 g 3-Amino-phenol
0,60 g 4-Hydroxy-1,2-methylendioxybenzol
0,10 g 3,5-Diamino-2,6-dimethoxy-pyridindihydrochlorid
0,40 g Natriumsulfit, wasserfrei
15,00 g Ölsäure
7,00 g Isopropanol
10,00 g Ammoniak (22 %ige wäßrige Lösung)
63,71 g Wasser
100,00 g

Man vermischt kurz vor der Anwendung 50 g dieses Haarfärbemittels in Gelform mit 50 ml einer 6 %igen Wasserstoffperoxidlösung. Das Gemisch wird sodann auf blonde menschliche Haare aufgetragen. Nach einer Einwirkungszeit von dreißig Minuten bei 40 Grad Celsius wird das Haar mit Wasser gespült und sodann getrocknet. Das Haar hat eine natürliche dunkelbraune Färbung angenommen.

### Beispiel 3: Haarfärbelösung

0,20 g 5-Fluor-2,4-bis[(2'hydroxyethyl)amino]-toluol
0,80 g p-Aminophenol
0,12 g Resorcin
0,10 g m-Aminophenol
1,05 g 5-Amino-2-methyl-phenol
0,40 g Natriumsulfit, wasserfrei
10,00 g Laurylalkohol-diglykolethersulfat (28 %ige wäßrige Lösung)
10,00 g Isopropanol
20,00 g Ammoniak (22 %ige wäßrige Lösung)
57,33 g Wasser, vollentsalzt
100,00 g

50 g der vorstehenden Haarfärbelösung werden kurz vor der Anwendung mit 50 ml einer 6 %igen Wasserstoffperoxidlösung vermischt. Das Gemisch wird sodann auf blonde menschliche Haare aufgetragen. Nach einer Einwirkungszeit von dreißig Minuten bei vierzig Grad Celsius wird das Haar mit Wasser gespült und getrocknet. Das Haar ist goldorange gefärbt.

**Beispiel 4: Haarfärbelösung**

0,61 g 5-Chlor-2,4-bis[(2'-hydoxyethyl)amino]-toluol
0,15 g 4-Amino-3-methyl-phenol
0,14 g 4-Amino-phenol
0,40 g Natriumsulfit, wasserfrei
10,00 g Laurylalkohol-diglykolethersulfat (28 %ige wäßrige Lösung)
10,00 g Isopropanol
20,00 g Ammoniak, vollentsalzt
58,70 g Wasser, vollentsalzt
100,00 g

50 g der vorstehenden Haarfärbelösung werden kurz vor der Anwendung mit 50 ml einer 6 %igen Wasserstoffperoxidlösung vermischt. Das Gemisch wird sodann auf blonde menschliche Haare aufgetragen. Nach einer Einwirkungszeit von dreißig Minuten bei vierzig Grad Celsius wird das Haar mit Wasser gespült und getrocknet. Das Haar ist in einem modischen Bordeauxrot-Ton gefärbt.

**Vergleichsbeispiele**

Zum Vergleich der Farbintensitäten im Rotbereich wurden als Farbstoffe jeweils eine der erfindungsgemäßen Kupplersubstanzen der allgemeinen Formel (I)
5-Fluor-2,4-bis(methylamino)-toluol
5-Fluor-2,4-bis(ethylamino)-toluol
5-Fluor-2,4-bis[(2'hydroxyethyl)amino]-toluol
5-Chlor-2,4-bis[(2'-hydroxyethyl)amino]-toluol
oder die bekannte Kupplersubstanz
2,4-Diamino-5-tetrafluorethoxy-toluol
in Kombination mit einer äquimolaren Menge einer der bekannten Entwicklersubstanzen
4-Amino-phenol (Beispiele I, III, V, VII, IX)
oder
4-Amino-3-methylphenol (Beispiele II, IV, VI, VIII, X)
in einer Haarfärbelösung eingesetzt.

**Beispiel I: Färbelösung**

10,000 g eines Gemisches aus den Kaliumsalzen der Kokosfettsäure und der Ölsäure
10,000 g Isopropanol
1,9125 g 5-Fluor-2,4-bis(methylamino)-toluol
1,3630 g 4-Aminophenol
10,0000 g Ammoniak (25 %ige wäßrige Lösung)
66,7245 g Wasser
100,0000 g

**Beispiel II: Färbelösung**

10,0000 g eines Gemisches aus dem Kaliumsalzen der Kokosfettsäure und der Ölsäure
10,0000 g Isopropanol
1,9125 g 5-Fluor-2,4-bis(methylamino)-toluol
1,5380 g 4-Amino-3-methylphenol
10,0000 g Ammoniak (25 %ige wäßrige Lösung)
66,5495 g Wasser
100,0000 g

**Beispiel III: Färbelösung**

10,0000 g eines Gemischs aus den Kaliumsalzen der Kokosfettsäure und der Ölsäure
10,0000 g Isopropanol
2,2625 g 5-Fluor-2,4-bis(ethylamino)-toluol
1,3630 g 4-Aminophenol

10,0000 g Ammoniak (25 %ige wäßrige Lösung)
66,3745 g Wasser
100,0000 g

**Beispiel IV: Färbelösung**

10,0000 g eines Gemischs aus den Kaliumsalzen der Kokosfettsäure und der Ölsäure
10,0000 g Isopropanol
2,2625 g 5-Fluor-2,4-bis(ethylamino)-toluol
1,5380 g 4-Amino-3-methylphenol
10,0000 g Ammoniak (25 %ige wäßrige Lösung)
66,1995 g Wasser
100,0000 g

**Beispiel V: Färbelösung**

10,0000 g eines Gemischs aus den Kaliumsalzen der Kokosfettsäure und der Ölsäure
10,0000 g Isopropanol
2,2625 g 5-Fluor-2,4-bis[(2'-hydroxyethyl)amino]-toluol
1,3630 g 4-Aminophenol
10,0000 g Ammoniak (25 %ige wäßrige Lösung)
66,3745 g Wasser
100,0000 g

**Beispiel VI: Färbelösung**

10,0000 g eines Gemischs aus den Kaliumsalzen der Kokosfettsäure und der Ölsäure
10,0000 g Isopropanol
2,2625 g 5-Fluor-2,4-bis[(2'-hydroxyethyl)amino]-toluol
1,5380 g 4-Amino-3-methylphenol
10,0000 g Ammoniak (25 %ige wäßrige Lösung)
66,1995 g Wasser
100,0000 g

**Beispiel VII: Färbelösung**

10,0000 g eines Gemischs aus den Kaliumsalzen der Kokosfettsäure und der Ölsäure
10,0000 g Isopropanol
2,8625 g 5-Chlor-2,4-bis[(2'-hydroxyethyl)amino]-toluol
1,3630 g 4-Aminophenol
10,0000 g Ammoniak (25 %ige wäßrige Lösung)
65,7745 g Wasser
100,0000 g

**Beispiel VIII: Färbelösung**

10,0000 g eines Gemischs aus den Kaliumsalzen der Kokosfettsäure und der Ölsäure
10,0000 g Isopropanol
2,8625 g 5-Chlor-2,4-bis[(2'-hydroxyethyl)amino]-toluol
1,5380 g 4-Amino-3-methylphenol
10,0000 g Ammoniak (25 %ige wäßrige Lösung)
65,5995 g Wasser
100,0000 g

**Beispiel IX: Färbelösung nach dem Stand der Technik**

10,0000 g eines Gemischs aus den Kaliumsalzen der Kokosfettsäure und der Ölsäure
10,0000 g Isopropanol

2,9625 g 2,4-Diamino-5-tetrafluorethoxy-toluol
1,3630 g 4-Aminophenol
10,0000 g Ammoniak (25 %ige wäßrige Lösung)
65,6745 g Wasser
100,0000 g

**Beispiel X: Färbelösung nach dem Stand der Technik**

10,0000 g eines Gemischs aus den Kaliumsalzen der Kokosfettsäure und der Ölsäure
10,0000 g Isopropanol
2,9625 g 2,4-Diamino-5-tetrafluorethoxy-toluol
1,5380 g 4-Amino-3-methylphenol
10,0000 g Ammoniak (25 %ige wäßrige Lösung)
65,4995 g Wasser
100,0000 g

Jeweils 5 g der Färbelösungen der Beispiele I bis X wurden kurz vor der Anwendung jeweils mit 5 g 6-prozentiger Wasserstoffperoxidlösung vermischt. Das Gemisch wurde sodann jeweils auf weiße Büffelhaarsträhnchen aufgetragen. Nach einer Einwirkungszeit von 30 Minuten bei 40 Grad Celsius wurden die Büffelhaarsträhnchen mit Wasser gespült und getrocknet.

Schließlich wurden die Farbmeßwerte der gefärbten Büffelhaarsträhnchen einem Farbmeßgerät (Chromameter CR 200 der Firma Minolta) bestimmt. Hierbei bedeutet Y die Helligkeit gegenüber einer rein weißen Oberfläche (Y = 100 %), das heißt je größer der Y-Wert ist, umso heller ist die Färbung.

Die Parameter x, y und z bedeuten den roten, grünen und blauen Anteil des jeweiligen Farbtons, wobei x und y gemessen werden und z sich von der Formel $z = 1-(x+y)$ errechnen läßt.

Die Farbmesswerte der gefärbten Büffelhaarsträhnchen sind in Tabelle 1 angegeben:

Tabelle 1

| **Farbmeßwerte** | | | | |
|---|---|---|---|---|
| Färbelösung | **Y** | **x** | **y** | **z** |
| Beispiel I | 5,5 | 0,417 | 0,303 | 0,280 |
| Beispiel III | 6,0 | 0,400 | 0,293 | 0,307 |
| Beispiel V | 6,8 | 0,394 | 0,287 | 0,319 |
| Beispiel VII | 9,3 | 0,382 | 0,297 | 0,321 |
| Beispiel IX | 35,8 | 0,355 | 0,333 | 0,312 |
| Beispiel II | 6,4 | 0,363 | 0,270 | 0,367 |
| Beispiel IV | 9,5 | 0,344 | 0,267 | 0,389 |
| Beispiel VI | 8,7 | 0,343 | 0,260 | 0,397 |
| Beispiel VIII | 14,1 | 0,340 | 0,278 | 0,382 |
| Beispiel X | 49,8 | 0,334 | 0,332 | 0,324 |

Die Vergleichsfärbungen zeigen, daß beim Färben mit den erfindungsgemäße Verbindungen (Kupplersubstanzen) der allgemeinen Formel (I) enthaltenden Färbelösungen (Beispiel I bis VIII) im Vergleich zu den nicht-erfindungsgemäße Kupplersubstanzen enthaltenden Färbelösungen (Beispiele IX und X) deutlich höhere Farbtiefen (= kleinere Y-Werte) erzielt werden.

**Beispiel A: Herstellung von 5-Fluor-2,4-bis(methylamino)-toluol**

Stufe 1: 5-Fluor-2,4-dinitro-toluol

5,5 g 3-Fluortoluol werden mit einem erkalteten Gemisch von 10,9 ml konzentrierter $H_2SO_4$ und 9,3 ml konzentrierter $HNO_3$ nitriert. Die Mischung wird 24 Stunden lang bei Raumtemperatur gerührt und anschliessend auf Eis gegossen. Das ausgefallene Produkt wird abgesaugt und mit Wasser gewaschen. Nach der Umkristallisation aus Ethanol werden 5,8 g (58 % der Theorie) schwach gelb gefärbte Kristalle mit einem Schmelzpunkt von 80 Grad Celsius erhalten.
$^1$H-NMR ($D_6$ -DMSO):

δ = 2,64 (s; 3 H, $-CH_3$)
7,85 (d, J = 12 Hz; 1 H, 6-H)
8,76 ppm (d, J = 7,1 Hz; 1 H, 3-H)
MS (70 eV): m/e = 200 ($M^+$)

Stufe 2: 5-Fluor-2,4-diamino-toluol

2 g (0,01 ml) 5-Fluor-2,4-dinitro-toluol aus Stufe 1 werden mit katalytischen Mengen Palladium/Kohlenstoff (10 % Pd) in 50 ml absolutem Ethanol hydriert. Nach dem Abfiltrieren des Katalysators wird das Lösungsmittel vollständig abdestilliert und das erhaltene Produkt aus Toluol umkristallisiert. Man erhält 0,7 g (50 % der Theorie) 5-Fluor-2,4-diamino-toluol, das bei 112 Grad Celsius unter Zersetzung schmilzt.
$^1$H-NMR ($D_6$ -DMSO):
δ = 2,49 (s; 3 H, $-CH_3$)
4,36 (s; 2 H, $NH_2$)
4,54 (s; 2 H, $NH_2$)
6,03 (d; J = 8,6 Hz, 3-H)
6,54 ppm (d; J = 12 Hz, 6-H)
MS (70 eV): m/e = 140 ($M^+$)

Stufe 3: 5-Fluor-2,4-bis[(N-benzolsulfonyl)amino]-toluol

8 g 5-Fluor-2,4-diamino-toluol aus Stufe 2 werden in 250 ml Pyridin gelöst, mit 7,9 ml Benzolsulfonylchlorid versetzt und 1 Stunde lang bei 120° Celsius gerührt. Nach dem Abkühlen wird der Reaktionsansatz auf Eis gegossen. Der erhaltene Niederschlag wird mit Wasser gewaschen, im Vakuum über Phosphorpentoxid getrocknet und aus Methanol umkristallisiert. Man erhält 3,9 g (41 % der Theorie) 5-Fluor-2,4-bis[(N-benzolsulfonyl)amino]-toluol, das bei 146° Celsius schmilzt.

Stufe 4: 5-Fluor-2,4-bis[(N-benzolsulfonyl,N-methyl)amino]-toluol

3 g 5-Fluor-2,4-bis[(N-benzolsulfonyl)amino]-toluol werden mit 0,9 g Kaliumhydroxid in 24 ml eines 1:1 Gemisches aus Wasser und Ethanol auf 30° Celsius erhitzt. Bei dieser Temperatur werden 1,78 ml Methyljodid zugegeben. Man rührt den Reaktionsansatz 2 Stunden lang bei 45° Celsius und gießt anschließend auf Eis. Der erhaltene Niederschlag wird abfiltriert und aus einem Ethanol-Wasser-Gemisch umkristallisiert. Man erhält 2,8 g (87 % der Theorie) 5-Fluor-2,4-bis [(N-benzolsulfonyl,N-methyl)amino]-toluol in Form beiger Kristalle mit einem Schmelzpunkt von 148° Celsius.

Stufe 5: 5-Fluor-2,4-bis(methylamino)-toluol

5 g 5-Fluor-2,4-bis[(N-benzolsulfonyl,N-methyl)amino]-toluol werden in einem Gemisch aus 20 ml Eisessig und 20 ml konzentrierter Schwefelsäure 20 Minuten lang bei 140° Celsius gerührt. Der Reaktionsansatz wird sodann auf Eis gegossen. Der pH-Wert wird mit wäßriger Natronlauge schwach alkalisch umgestellt und der erhaltene Niederschlag abgesaugt. Man erhält nach dem Trocknen des Niederschlags 1,2 g 5-Fluor-2,4-bis (methylamino)-toluol (62 % der Theorie) mit einem Schmelzpunkt von 60° Celsius.
$^1$H-NMR ($D_6$-DMSO):
δ = 1,93 (s; 3 H, $-CH_3$),
2,50 (s; 3 H, $-NCH_3$),
2,81 (s; 3 H, $-NCH_3$),
4,61 (br. s; 1 H, -NH, tauscht mit $D_2O$ aus),
4,96 (br. s; 1 H, -NH, tauscht mit $D_2O$ aus),
5,79 (d, J = 8,1 Hz; 1 H, 3-H) und
6,63 ppm (d, J = 12,4 Hz; 1 H, 6-H)
MS (70eV): m/e = 168 ($M^+$)

**Beispiel B: Herstellung von 5-Fluor-2,4-bis(ethylamino)-toluol**

Stufen 1, 2 und 3: 5-Fluor-2,4-bis[(N-benzolsulfonyl)amino]-toluol

5-Fluor-2,4-bis[(N-benzolsulfonyl)amino]-toluol wird gemäß den beim Beispiel A beschriebenen Stufen 1, 2 und 3 aus 3-Fluortoluol hergestellt.

Stufe 4: 5-Fluor-2,4-bis[(N-benzolsulfonyl-N-ethyl)amino]-toluol

5 g 5-Fluor-2,4-bis[(N-benzolsulfonyl)amino]-toluol aus Stufe 3 werden mit 0,9 g Kaliumhydroxid in 24 ml eines 1:1 Gemisches aus Wasser und Ethanol auf 30° Celsius erhitzt. Bei dieser Temperatur werden 3,22 ml Ethyljodid zugegeben. Man rührt den Reaktionsansatz 2 Stunden lang bei 55° Celsius und gießt ihn anschließend auf Eis. Der erhaltene Niederschlag wird abfiltriert und aus einem Ethanol-Wasser-Gemisch umkristallisiert. Man erhält 2,6 g (46% der Theorie) 5-Fluor-2,4-bis[(N-benzolsulfonyl-N-ethyl)amino]-toluol in Form beiger Kristalle mit einem Schmelzpunkt von 120° Celsius.

Stufe 5: 5-Fluor-2,4-bis(ethylamino)-toluol

2 g 5-Fluor-2,4-bis[(N-benzolsulfonyl,N-ethyl)amino]-toluol werden in einem Gemisch aus 8 ml Eisessig und 8 ml konzentrierter Schwefelsäure 20 Minuten lang bei 140° Celsius gerührt. Der Reaktionsansatz wird sodann auf Eis gegossen. Der pH-Wert wird mit wäßriger Natronlauge schwach alkalisch eingestellt und der erhaltene Niederschlag abgesaugt. Man erhält nach dem Trocknen 0,4 g 5-Fluor-2,4-bis(ethylamino)-toluol (54 % der Theorie) in Form farbloser Kristalle mit einem Schmelzpunkt von 50° Celsius.
$^1$H-NMR ($D_6$-DMSO):
δ = 1,16 (t, J = 7,4 Hz; 3 H, $-CH_2-CH_3$),
1,18 (t, J = 7,4 Hz; 3 H, $-CH_2-CH_3$),
1,94 (s; 3 H, $-CH_3$)
3,03 - 3,09 (q, J = 6 Hz; 4 H, $-CH_2-CH_3$),
4,23 (br. s; 1 H, -NH, tauscht mit $D_2O$ aus),
4,68 (br. s; 1 H, -NH, tauscht mit $D_2O$ aus),
5,87 (d, J = 8,1 Hz; 1 H, 3-H) und
6,39 ppm (d, J = 12,4 Hz; 1 H, 6-H)
MS (70ev): m/e = 196 ($M^+$)

**Beispiel C: Herstellung von 5-Fluor-2,4-bis[(2'-hydroxyethyl)amino]-toluol**

Stufen 1, 2: 5-Fluor-2,4-diamino-toluol

Die Herstellung von 5-Fluor-2,4-diamino-toluol erfolgt gemäß den bei Beispiel A beschriebenen Stufen 1 und 2 aus 3-Fluortoluol.

Stufe 3: 5-Fluor-2,4-bis-[(2'-hydroxyethyl)amino]-toluol

0,56 g 5-Fluor-2,4-diamino-toluol werden mit 0,66 g Kalziumcarbonat in 20 ml Dioxan auf 70° Celsius erhitzt, mit 1 ml Chlorameisensäurechlorethylester versetzt und 3 Stunden lang bei 100° Celsius erhitzt. Sodann wird heiß filtriert und das gelbe Filtrat auf 200 ml Eiswasser gegeben. Der entstandene Niederschlag wird abfiltriert und aus einem Ethanol-Wasser-Gemisch umkristallisiert.

0,5 g des umkristallisierten Niederschlags werden mit einer Mischung aus 0,42 g Kaliumhydroxid in 5,6 ml Ethanol und 2,4 ml Wasser versetzt und 1 Stunde lang unter Rückfluß erhitzt. Nach dem Abkühlen wird die Lösung mit Wasser verdünnt, mit Essigsäure neutralisiert und mit Essigester extrahiert.

Durch Chromatographie an Kiesgel 60 mit einer Korngröße von 0,5 bis 2 mm mit einem 1:1 Gemisch aus Chloroform und Methanol und Einengen des Eluats werden 0,21 g 5-Fluor-2,4-bis[(2'-hydroxyethyl)-amino)]-toluol (35 % der Theorie) in Form schwarzer Kristalle mit einem Schmelzpunkt von 88° Celsius erhalten.
$^1$H-NMR ($D_6$-DMSO):
δ = 1,95 (s; 3 H, $-CH_3$),
3,10 - 3,14 (m; 4 H, $-CH_2-OH$),
3,59 (m; 4 H, $-NH-CH_2$),

4,25 (br.s; 1 H, -OH, tauscht mit $D_2O$ aus),
4,65 (br.s; 1 H, -OH, tauscht mit $D_2O$ aus),
4,72 (br.s; 2 H, -NH, tauscht mit $D_2O$ aus),
5,95 (d, J = 8,1 Hz; 1 H, 3-H) und
6,65 ppm (d, J = 12,3 Hz; 1 H, 6-H)
MS (70ev) : m/e = 228 ($M^+$)

**Beispiel D: Herstellung von 5-Chlor-2,4-bis[(2'-hydroxyethyl)amino]-toluol**

Stufe 1: 5-Chlor-2,4-diamino-toluol

5-Chlor-2,4-diamino-toluol wird mit dem der bei F. Reverdin, P. Crépieux, Chemische Berichte 33, (1900), 2505 bis 2508 beschriebenen Verfahren hergestellt.

Stufe 2: 5-Chlor-2,4-bis[(2'-hydroxyethyl)amino]-toluol

0,8 g 5-Chlor-2,4-diamino-toluol werden mit 0,85 g Kalziumcarbonat in 26 ml Dioxan auf 70 ° Celsius erhitzt, mit 1,3 ml Chlorameisensäureethylester versetzt und 3 Stunden lang bei 100 ° Celsius gerührt. Sodann wird heiß filtriert und das gelbe Filtrat wird auf 256 ml Eiswasser gegeben. Der entstandene Niederschlag wird abfiltriert und aus einem Ethanol-Wasser-Gemisch umkristallisiert.

0,64 g des umkristallisierten Niederschlags werden mit einer Mischung aus 0,54 g Kaliumhydroxid in 7,2 ml Ethanol und 3 ml Wasser versetzt und 1 Stunde lang unter Rückfluß erhitzt. Nach dem Abkühlen wird die Lösung mit Wasser verdünnt, mit Essigsäure neutralisiert und mit Essigester extrahiert.
Durch Chromatrographie des Essigesterextraktes an Kieselgel 60 mit einer Korngröße von 0,5 bis 2 mm mit einem 1:1 Gemisch aus Chloroform und Methanol und anschließendes Einengen des Eluats werden 0,12 g 5-Chlor-2,4-bis[(2'-hydroxyethyl)amino]-toluol (18 % der Theorie) in Form brauner Kristalle mit einem Schmelzpunkt von 83 ° Celsius erhalten.
$^1$H-NMR ($D_6$-DMS0):
$\delta$ = 1,94 (s; 3H, $-CH_3$),
3,57 - 3,62 (m; 4H, $-NH-CH_2-$),
4,58 (t, J = 5,5 Hz; 1H; -OH, tauscht mit $D_20$ aus),
4,67 (t, J = 5,6 Hz; 1H, -OH, tauscht mit $D_20$ aus),
4,75 (t, J = 5,5 Hz; 1H; -NH, tauscht mit $D_20$ aus),
4,80 (t, J = 5,3 Hz; 1H; -NH, tauscht mit $D_2$ aus)
5,92 (s; 1H, 3-H) und
6,82 ppm (s; 1H, 6-H)
MS(70eV): m/e = 244 ($M^+$)
Alle in der vorliegenden Anmeldung angegebenen Prozentzahlen stellen, sofern nicht anders vermerkt, Gewichtsprozent dar.

**Patentansprüche**

1. Mittel zur oxidativen Färbung von Haaren auf der Basis einer Entwicklersubstanz-Kupplersubstanz-Kombination sowie gegebenenfalls anderer Farbkomponenten und in Haarfärbemitteln üblichen Zusätzen, dadurch gekennzeichnet, daß es mindestens eine Kupplersubstanz der allgemeinen Formel

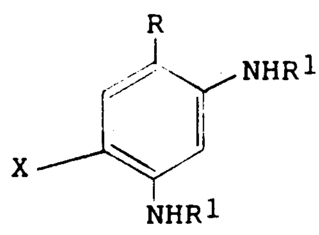

(I),

wobei R einen geradkettigen oder verzweigten $C_1$- bis $C_4$-Alkylrest bedeutet, X Chlor oder Fluor und $R^1$ einen geradkettigen oder verzweigten $C_1$- bis $C_4$-Alkylrest oder einen geradkettigen oder verzweigten $C_2$- bis $C_4$-Hydroxyalkylrest darstellt, oder

deren physiologisch verträgliches wasserlösliches Salz enthält.

**2.** Mittel nach Anspruch 1, dadurch gekennzeichnet, daß es die Kupplersubstanz der allgemeinen Formel (I) in einer Menge von 0,01 bis 5 Gewichtsprozent enthält.

**3.** Mittel nach Anspruch 1 oder 2, dadurch gekennzeichnet, daß es als Kupplersubstanz 5-Fluor-2,4-bis-(methylamino)-toluol, 5-Fluor-2,4-bis(ethylamino)-toluol, 5-Fluor-2,4-bis[(2'-hydroxyethyl)amino]-toluol oder 5-Chlor-2,4-bis[(2'-hydroxyethyl)amino]-toluol enthält.

**4.** Mittel nach einem der Ansprüche 1 bis 3, dadurch gekennzeichnet, daß es zusätzlich mindestens eine weitere Kupplersubstanz enthält, welche ausgewählt ist aus Resorcin, 4-Chlorresorcin, 4,6-Dichlorresorcin, 2-Methylresorcin, 2-Amino-4-(2'-hydroxyethylamino)anisol, 2-Amino-4-ethylaminoanisol, 2,4-Diaminobenzylalkohol, m-Phenylendiamin, 5-Amino-2-methylphenol, 2,4-Diaminophenoxyethanol, 1-Naphthol, m-Aminophenol, 3-Amino-4-chlor-6-methylphenol, 3-Amino-2-methyl-phenol, 4-Amino-2-hydroxyphenoxyethanol, 4-Hydroxy-1,2-methylendioxybenzol, 4-(2'-Hydroxyethylamino)-1,2-methylendioxybenzol, 2,4-Diamino-5-ethoxytoluol, 4-Hydroxyindol, 3-Amino-5-hydroxy-2,6-dimethoxypyridin und 3,5-Diamino-2,6-dimethoxypyridin.

**5.** Mittel nach einem der Ansprüche 1 bis 4, dadurch gekennzeichnet, daß die Entwicklersubstanz ausgewählt ist aus 1,4-Diaminobenzol, 2,5-Diaminotoluol, 2,5-Diaminobenzylalkohol, 2-(2'-Hydroxyethyl)-1,4-diaminobenzol, 4-Aminophenol, 4-Amino-2-aminomethyl-phenol, 4-Amino-3-methylphenol, 4-Amino-2-methoxymethylphenol, 4-Amino-2-ethoxymethylphenol sowie Tetraaminopyrimidin oder deren physiologisch verträglichen Salzen.

**6.** Mittel nach einem der Ansprüche 1 bis 5, dadurch gekennzeichnet, daß die Gesamtmenge der Entwicklersubstanz-Kupplersubstanz-Kombination 0,1 bis 5,0 Gewichtsprozent beträgt.

**7.** Mittel nach einem der Ansprüche 1 bis 6, dadurch gekennzeichnet, daß es eine Farbkomponente enthält, welche ausgewählt ist aus 6-Amino-2-methylphenol, 2-Amino-5-methylphenol, Basic Violet 14 (C. I. 42 510), Basic Violet 2 (C. I. 42 520), 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethylamino)-nitrobenzol, 4-(2'-Hydroxyethylamino)-3-nitrotoluol, 1-(2'-Ureidoethyl)-amino-4-nitrobenzol, Acid Brown 4 (C. I. 14 805), 1,4-Diaminoanthrachinon und 1,4,5,8-Tetraaminoanthrachinon.

**8.** 5-Halogen-2,4-bis(alkylamino)-1-alkylbenzol der allgemeinen Formel

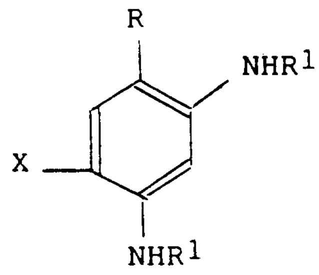

(I),

wobei R einen geradkettigen oder verzweigten $C_1$-bis $C_4$-Alkylrest bedeutet, X Chlor oder Fluor und $R^1$ einen geradkettigen oder verzweigten $C_1$- bis $C_4$-Alkylrest oder einen geradkettigen oder verzweigten $C_2$- bis $C_4$-Hydroxyalkylrest darstellt.

**9.** 5-Fluor-2,4-bis(methylamino)-toluol

**10.** 5-Fluor-2,4-bis(ethylamino)-toluol

**11.** 5-Fluor-2,4-bis[(2'-hydroxyethyl)amino]-toluol

**12.** 5-Chlor-2,4-bis[(2'-hydroxyethyl)amino]-toluol

## Claims

1. Agent for the oxidative colouring of hair based on a developer substance-coupler substance combination and optionally other colouring components and additives conventional in hair colouring agents, characterised in that it contains at least one coupler substance of the general formula

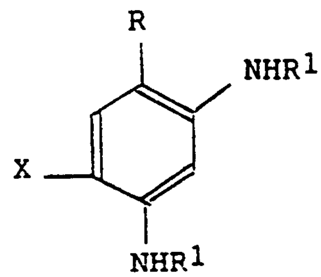

(I),

in which R signifies a straight chain or branched $C_1$-$C_4$ alkyl residue, X is chlorine or fluorine and $R^1$ is a straight chain or branched $C_1$-$C_4$ alkyl residue or a straight chain or branched $C_2$ to $C_4$ hydroxy alkyl residue, or their physiologically compatible water-soluble salt.

2. Agent according to Claim 1, characterised in that it contains the coupler substance of the general formula (I) in an amount of from 0.01 to 5 weight %.

3. Agent according to Claim 1 or Claim 2, characterised in that it contains 5-fluoro-2,4-bis(methylamino)-toluene, 5-fluoro-2,4-bis(ethylamino)-toluene, 5-fluoro-2,4-bis[(2'-hydroxyethyl)-amino]-toluene or 5-chloro-2,4-bis[(2'-hydroxy-ethyl)amino]-toluene as coupler substance.

4. Agent according to any one of Claims 1 to 3, characterised in that it additionally contains at least one further coupler substance which is selected from resorcinol, 4-chlororesorcinol, 4,6-dichlororesorcinol, 2-methylresorcinol, 2-amino-4-(2'-hydroxyethylamino)anisole, 2-amino-4-ethylamino-anisole, 2,4-diaminobenzylalcohol, m-phenylenediamine, 5-amino-2-methylphenol, 2,4-diaminophenoxy-ethanol, 1-naphthol, m-aminophenol, 3-amino-4-chloro-6-methylphenol, 3-amino-2-methylphenol, 4-amino-2-hydroxyphenoxyethanol, 4-hydroxy-1,2-methylenedioxybenzene, 4-(2'-hydroxyethylamino)-1,2-methylenedioxybenzene, 2,4-diamino-5-ethoxytoluene, 4-hydroxyindole, 3-amino-5-hydroxy-2,6-dimethoxypyridine and 3,5-diamino-2,6-dimethoxypyridine.

5. Agent according to any one of Claims 1 to 4, characterised in that the developer substance is selected from 1,4-diaminobenzene, 2,5-diaminotoluene, 2,5-diaminobenzylalcohol, 2-(2'-hydroxy-ethyl)-1,4-diaminobenzene, 4-aminophenol, 4-amino-2-aminomethyl-phenol, 4-amino-3-methylphenol, 4-amino-2-methoxymethylphenol, 4-amino-2-ethoxy-methylphenol and tetraaminopyrimidine or their physiologically compatible salts.

6. Agent according to any one of Claims 1 to 5, characterised in that the total amount of the developer substance-coupler substance combination is from 0.1 to 5.0 weight %.

7. Agent according to any one of Claims 1 to 6, characterised in that it contains a colouring component which is selected from 6-amino-2-methylphenol, 2-amino-5-methylphenol, Basic Violet 14 (C.I. 42 510), Basic Violet 2 (C.I. 42 520), 2-nitro-1,4-diaminobenzene,2-amino-4-nitrophenol, 2-amino-5-nitrophenol, 2-amino-4,6-dinitrophenol, 2-amino-5-(2'-hydroxyethylamino)-nitrobenzene, 4-(2'-hydroxyethylamino)-3-nitrotoluene, 1-(2'-ureidoethyl)-amino-4-nitrobenzene, Acid Brown 4 (C.I. 14 805), 1,4-diaminoanthraquinone and 1,4,5,8-tetraaminoanthraquinone.

**8.** 5-halogen-2,4-bis(alkylamino)-1-alklybenzene of the general formula

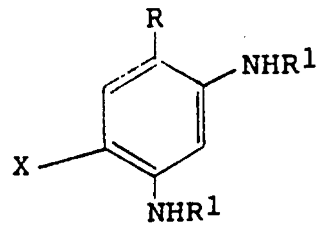

(I),

in which R signifies a straight chain or branched $C_1$ to $C_4$ alkyl residue, X is chlorine or fluorine and $R^1$ is a straight chain or branched $C_1$ to $C_4$ alkyl residue or a straight chain or branched $C_2$ to $C_4$ hydroxy alkyl residue.

**9.** 5-fluoro-2,4-bis(methylamino)-toluene.

**10.** 5-fluoro-2,4-bis(ethylamino)-toluene.

**11.** 5-fluoro-2,4-bis[(2'-hydroxyethyl)amino]-toluene.

**12.** 5-chloro-2,4-bis[(2'-hydroxyethyl)amino]-toluene.

## Revendications

**1.** Agent pour la coloration par oxydation des cheveux sur la base d'une combinaison d'une substance de développement et d'une substance de couplage ainsi qu'éventuellement d'autres composants colorés et d'adjuvants habituels d'agents de coloration des cheveux, caractérisé en ce qu'il comprend au moins une substance de couplage de formule générale

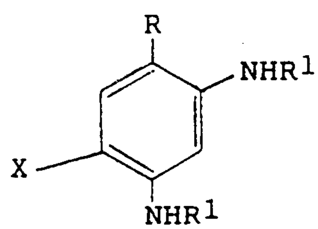

(I),

où R représente un radical alkyle $C_1$ à $C_4$ à chaîne rectiligne ou à branches, X du chlore ou du fluor et $R^1$ un radical alkyle $C_1$ à $C_4$ à chaîne rectiligne ou à branches ou un radical hydroxyalkyle $C_2$ à $C_4$ à chaîne rectiligne ou à branches, ou leur sel soluble dans l'eau et physiologiquement compatible.

**2.** Agent selon la revendication 1, caractérisé en ce qu'il contient la substance de couplage de formule générale (I) selon une quantité de 0,01 à 5% en poids.

**3.** Agent selon la revendication 1 ou 2, caractérisé en ce qu'il contient en tant que substance de couplage: 5-fluoro-2,4-bis(méthylamino)-toluène, 5-Fluoro-2,4-bis(éthylamino)-toluène, 5-fluoro-2,4-bis[(2'-hydroxyéthyl)-amino]-toluène ou 5-chloro-2,4-bis [(2'-hydroxyéthyl)amino]-toluène.

**4.** Agent selon l'une des revendications 1 à 3, caractérisé en ce qu'il contient en outre au moins une autre substance de couplage, qui est choisie parmi: résorcine, 4-chlororésorcine, 4,6-dichlororésorcine, 2-méthylrésorcine, 2-amino-4-(2'-hydroxyéthylamino)anisol, 2-amino-4-éthylaminoanisol, 2,4-diaminobenzylalcool, m-phénylènediamine, 5-amino-2-méthylphénol, 2,4-diaminophénoxyéthanol, 1-naphtol, m-aminophénol, 3-amino-4-chloro-6-méthylphénol, 3-amino-2-méthyl-phénol, 4-amino-2-hydroxyphénoxyéthanol, 4-hydroxy-1,2-methylènedioxybenzène,4-(2'-hydroxyéthylamino)-1,2-méthylènedioxybenzène, 2,4-diamino-5-éthoxytoluène, 4-hydroxyindol, 3-amino-5-hydroxy-2,6-diméthoxypyridine, et 3,5-diamino-2,6-diméthoxypyridine.

5. Agent selon l'une des revendications 1 à 4, caractérisé en ce que la substance de développement est choisie parmi: 1,4-diaminobenzène, 2,5-diaminotoluène, 2,5-diaminobenzylalcool, 2-(2'-hydroxyéthyl)-1,4-diaminobenzène, 4-aminophénol, 4-amino-2-aminométhylphénol, 4-amino-3-méthylphénol, 4-amino-2-méthoxyméthylphénol, 4-amino-2-éthoxy-méthylphénol, ainsi que tétraaminopyrimidine ou leurs sels physiologiquement compatibles.

6. Agent selon l'une des revendications 1 à 5, caractérisé en ce que la quantité totale de la combinaison formée par la substance de développement et par la substance de couplage est de 0,1 à 5,0 % en poids.

7. Agent selon l'une des revendications 1 à 6, caractérisé en ce qu'il contient un composant coloré qui est choisi parmi: 6-amino-2-méthylphénol 2-amino-5-méthylphénol Basic Violet 14 (C. I. 42 510), Basic Violet 2 (C. I. 42 520), 2-nitro-1,4-diaminobenzène, 2-amino-4-nitrophénol 2-amino-5-nitrophénol 2-amino-4,6-dinitrophénol, 2-amino-5-(2'-hydroxyéthylamino)nitrobenzène, 4-(2'-hydroxyéthylamino)-3-nitrotoluène, 1-(2'-uréidoéthyl)-amino-4-nitrobenzène, Acid Brown 4 (C.I. 14 805), 1'4-diaminoianthrachinone et 1,4,5,8-tétraaminoanthrachinone.

8. 5-halogène-2,4-bis(alkylamino)-1-alkylbenzol de formule générale

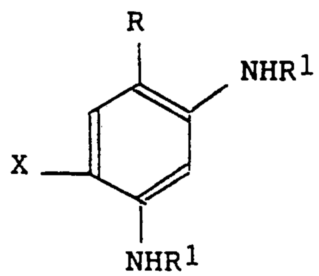

(I),

où R représente un radical alkyle $C_1$ à $C_4$ à chaîne rectiligne ou à branches, X du chlore ou du fluor et $R^1$ un radical alkyle $C_1$ à $C_4$ à chaîne rectiligne ou à branches ou un radical hydroxyalkyle $C_2$ à $C_4$ à chaîne rectiligne ou à branches.

9. 5-fluor-2,4-bis(méthylamino)-toluène.

10. 5-fluor-2,4-bis(éthylamino)-toluène.

11. 5-fluor-2,4-bis[(2'-hydroxyéthyl)amino]-toluène.

12. 5-chloro-2,4-bis[(2'-hydroxyéthyl)amino]-toluène.